# EUROPEAN PATENT SPECIFICATION

(11) **EP 2 164 492 B1**
(45) Date of publication and mention of the grant of the patent: **04.01.2012**
(21) Application number: 08762654.5
(22) Date of filing: 19.05.2008
(51) Int. Cl.: A61K 31/495, A61P 25/18, A61K 45/06

(54) **Trans-4-{2-[4-(2,3-dichlorophenyl)-piperazin-1-yl]-ethyl}-N,N-dimethylcarbamoyl-cyclohexylamine for treating schizophrenia**
Trans-4-{2-[4-(2,3-dichlorophenyl)-piperazin-1-yl]-ethyl}-N,N-dimethylcarbamoyl-cyclohexylamine zur Behandlung von Schizophrenie
Trans-4-{2-[4-(2,3-dichlorophenyl)-piperazin-1-yl]-ethyl}-N,N-dimethylcarbamoyl-cyclohexylamine pour le traitement de la schizophrénie

(30) Priority: 24.05.2007 HU 0700369
(43) Date of publication of application: 24.03.2010
(73) Proprietor: Richter Gedeon Nyrt., 1103 Budapest (HU)
(72) Inventor: LASZLOVSZKY, István, H-1111 Budapest (HU); NÉMETH, György, H-1021 Budapest (HU); ANDOR, György, H-1118 Budapest (HU)
(74) Representative: HOFFMANN EITLE
(86) International application number: PCT/HU2008/000051
(87) International publication number: WO 2008/142462

(56) References cited:
- WO-A-03/029233
- WO-A-2005/012266
- WO-A1-2008/141135
- WO-A1-2008/141135
- "Schizophrenia and related disorders" In: Mark H. Beers et al: "The Merck Manual, 18th edition", 19 May 2010 (2010-05-19), Merck Research Laboratories, US ISBN: 0911910182 pages 1722-1730,
- "Schizophrenia and related disorders" In: Mark H. Beers et al: "The Merck Manual, 18th edition", 19 May 2010 (2010-05-19), Merck Research Laboratories, US ISBN: 0911910182 pages 1722-1730,
- Use of cariprazine in the treatment of schizophrenia: a proof-of-concept trial, Robert Litman et al.; Forest Laboratories, Inc.

## Description

### FIELD OF THE INVENTION

The present invention relates to trans-4-{2-[4-(2,3-dichlorophenyl)-piperazin-1-yl]-ethyl}-N,N-dimethylcarbamoyl-cyclohexylamine and pharmaceutically acceptable salts thereof for use in a method of treating schizophrenia with the therapeutically effective amount of 1.5, 3.0 or 4.5 mg/day.

### BACKGROUND OF THE INVENTION

Schizophrenia is a lifelong disabling psychiatric disorder with a reported worldwide prevalence of about 1%, including 3.2 million Americans (see, e.g., National Institute of Mental Health, Schizophrenia, http://www.nimh.mh-gov/healthinformation/schizophreniamenu.cfm 2006; Mueser and McGurk, Lancet 2004;363:2063-72, 2004). The disorder usually manifests during adolescence or in young adulthood; the cardinal symptoms fall into three domains - positive symptoms, such as delusions and hallucinations, negative symptoms, such as lack of drive and social withdrawal, and cognitive symptoms, such as problems with attention and memory. These lead to social and occupational dysfunction, which inevitably have a profound effect on the family and the place of the affected individual in wider society. In addition to psychiatric symptoms, patients with schizophrenia are at greater risk for medical comorbidities than the general population.

Drug treatment with dopamine antagonists is the cornerstone of schizophrenia management both during the acute as well as the residual phase. Current guidelines recommend atypical antipsychotics, including risperidone, olanzapine, quetiapine, ziprasidone, and aripiprazole, as first-line treatment for schizophrenia. These drugs can be uniformly characterized by their dual mode of action: in addition to antagonism of the dopamine D₂ receptor, they are also potent inhibitors at the serotonin 5-HT_{2A} receptor.

Although an improvement over the classical neuroleptics, atypical antipsychotics still have shortcomings in the effective management of the disease. In particular, these drugs are associated with a high incidence of side effects (eg extrapyramidal symptoms [EPSs] at high dose, sedation, cardiovascular effects such as QTc prolongation, hematologic alterations, effects on sexual function, weight gain, metabolic abnormalities). Furthermore, treatment resistance remains high with 10% to 30% of patients having little or no response to currently available antipsychotic medications, and up to an additional 30% of patients having only partial treatment response (see, e.g., Lehman et al., Am. J. Psychiatry,161(2 Suppl), 1-56, 2004). This has led to the common clinical practice of experimental use of high doses of atypicals, antipsychotic polypharmacy, and augmentation with other psychotropic drugs (see, e.g., Zink et al., Eur. Psychiatry, 19:56-58, 2004; Stahl and Grady, Curr. Med. Chem., 11, 313-27, 2004).

According to the American Psychiatric Association guidelines for the treatment of schizophrenia, 60 % to 70 % of patients relapse within 1 year without maintenance treatment and almost 90% relapse within 2 years (see, e.g., Lehman et al., Am. J. Psychiatry, 161 (2 Suppl), 1-56, 2004).

Overall, a significant unmet medical need in the treatment of schizophrenia still exists and much effort is being made to identify and develop improved antipsychotic agents.

In this search, the D₃ dopamine receptor has emerged as a possible target for antipsychotic drug treatment. This strategy is based on the brain distribution and suggested role of D₃ receptors, which have the highest density in the ventral striatum (see, e.g., Gurevich and Joyce, Neuropsychopharmacology, 20, 60-80, 1999), one of the core areas in the disease pathology. Dopamine D₃ receptors appear to participate in regulation of motor activity (see, e.g., Shafer and Levant, Psychopharmacology (Berl), 135, 1-1, 1998) and cognitive function (see, e.g., Ukai et al., Eur. J. Pharmacol., 324, 147-51, 1997; Smith et al., Pharmacol. Biochem. Behav., 63, 201-11, 1999). Selective antagonists of the receptor modulated or even abolished motor disturbances (catalepsy) induced by D₂ antagonists (see, e.g., Millan et al., Eur. J. Pharmacol., 321, R7-9, 1997; Gyertyán et al., [abstract]. Int. J. Neruopsychopharmacol., 5 Suppl. 1, 174, 2002) in rodent models and did not cause motor side effects or increase prolactin levels when given alone (see, e.g., Reavill et al., A. J. Pharmacol. Exp. Ther., 294, 1154-65, 2000; Millan et al., J. Pharmacol. Exp. Ther., 293, 1063-73, 2000). This suggests greatly reduced extrapyramidal side effect liability. D₃ antagonists were shown to improve cognitive deficiencies caused by various agents in rodents (see, e.g., Sigala et al., Eur. J. Pharmacol., 336, 107-12, 1997; Laszy et al., Psychopharmacology (Berl), 179, 567-75, 2005) and primates, suggesting the possibility of a promising approach to the treatment of cognitive disturbances in schizophrenia. Dopamine D₃ receptor antagonists increase the motor activity of animals in habituated environments (see, e.g., Waters et al., J. Neural. Transm. Gen. Sect., 98, 39-55, 1994; Sautel et al., J. Pharmacol. Exp. Ther., 275, 1239-46, 1995; Gyertyán and Sághy, Behav. Pharmacol., 15, 253-62, 2004). This activating effect may prove to be beneficial against the negative symptoms of the disease.

However, the lack of effect of selective D₃ antagonists in various animal models used so far to assess (human) antipsychotic action suggests that "pure" D₃ antagonists alone may not have sufficiently robust antipsychotic effects to justify clinical development. It has been shown that approximately 60 % to 80 % occupancy of dopamine D₂ receptors is necessary to achieve an antipsychotic action in the clinic (see, e.g., Nyberg et al., Br. J. Psychiatry. Suppl., (29), 40-4, May 1996; Seeman, Clin. Neurosci. Res., 1,53-60,2001). This latter phenomenon provides the basis for the view that D₂ antagonism is essential for antipsychotic action. Therefore, it is considered that addition of D₃ antagonism to D₂ antagonism may offer distinct advantages over the existing antipsychotics in the treatment of schizophrenia, namely no EPSs, cognitive enhancing potential, and augmented effect on negative symptoms.

U.S. Patent Publication No. 2006/0229297 discloses (thio)-carbamoyl-cyclohexane derivatives that are D₃ and D₂ dopamine receptor subtype preferring ligands, having the formula (I): 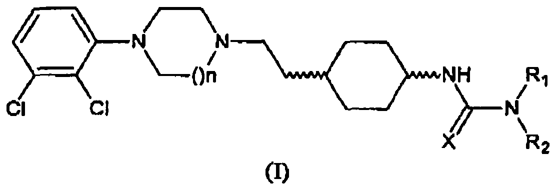 wherein R₁, R₂, X, and n are as defined therein.

Hungarian Patent Application No. P0700339 discloses salts of trans-4-{2-[4-(2,3-dichlorophenyl)-piperazin-1-yl]-ethyl}-N,N-dimethylcarbamoyl-cyclohexylamine. One particular compound disclosed therein is trans-4-{2-[4-(2,3-dichloiophenyl)-piperazin-1-yl]-ethyl}-N,N-dimethylcarbmoyl-cyclohexylamine hydrochloride, which is also known as trans-1 {4-[2-[4-(2,3-dichlorophenyl)-piperazin-1-yl]-ethyl]-cyclohexyl}-3,3-dimethyl-urea hydrochloride, the structural formula for which is shown below:

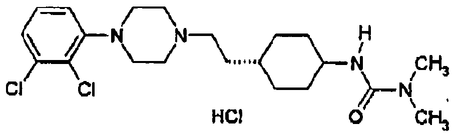

Compounds of US2006/0229297 are used for the treatment of schizophrenia, schizo-affective disorders at dosage ranges from 1 to 500 mg or 0.1 to 100 mg, given 1 to 4 times per day.

These (thio)-carbamoyl-cyclohexane derivatives are orally active and very potent dopamine D₃/D₂ receptor antagonists, which bind with significantly higher potency to D₃ than D₂ receptors. The D₃ receptor antagonism is about one order of magnitude greater than the D₂ receptor antagonism, which is believed to counteract some of the extrapyramidal side effects produced by D₂ receptor antagonists. In addition to the increased relative affinity for dopamine D₃ to D₂, e.g. trans-4-{2-[4-(2,3-dichlorophenyl)-piperazin-1-yl]-ethyl}-N,N-dimethylcarbamoyl-cyclohexylamine hydrochloride has a low potency at other receptor sites such as the 5-HT_{2C}, histamine H₁, and adrenergic receptor sites, which suggest a lower potential for side effects such as EPSs and body weight gain.

Thus, there is an existing and continual need for effective treatments for the symptoms of schizophrenia and cognitive effects associated with schizophrenia, without some of the side effects associated with traditional treatments.

WO 2008/141135, representing state of the art pursuant to Article 54(3) EPC, concerns solvate and crystalline forms of trans-1-{4-[2-[4-(2,3-dichlorophenyl)-piperazin-1-yl]-ethyl]-cyclohexyl}-3,3-dimethyl-urea hydrochloride and their use for treating conditions such as schizophrenia, schizo-affective disorders, wherein the dosage ranges from 1 to 500 mg or 0.1 to 100 mg daily, given in 1 to 4 doses, depending on the method of administration.

### SUMMARY OF THE INVENTION

The present invention relates to trans-4-{2-[4-(2,3-dichlorophenyl)-piperazin-1-yl]-ethyl}-N,N-dimethylcarbamoyl-cyclohexylamine and pharmaceutically acceptable salts thereof for use in a method of treating schizophrenia with the therapeutically effective amount of 1.5, 3.0 or 4.5 mg/day.

### DETAILED DESCRIPTION OF THE INVENTION

The present invention relates to trans-4-{2-[4-(2,3-dichlorophenyl)-piperazin-1-yl]-ethyl}-N,N-dimethylcarbamoyl-cyclohexylamine and/or salts and/or hydrates and/or solvates and/or polymorphs thereof in a for use in a method of treating schizophrenia with the therapeutically effective amount of 1.5, 3.0 or 4.5 mg/day.

Pharmaceutically acceptable salts include those obtained by reacting the main compound, functioning as a base with an inorganic or organic acid to form a salt, for example, salts of hydrochloric acid, sulfuric acid, phosphoric acid, methane sulfonic acid, camphor sulfonic acid, oxalic acid, maleic acid, succinic acid, citric acid, formic acid, hydrobromic acid, benzoic acid, tartaric acid, fumaric acid, salicylic acid, mandelic acid, and carbonic acid. Pharmaceutically acceptable salts also include those in which the main compound functions as an acid and is reacted with an appropriate base to form, e.g., sodium, potassium, calcium, magnesium, ammonium, and choline salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts can be prepared by reacting the compounds of the invention with the appropriate base via a variety of known methods.

The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fumarates, hydrobromides, hydroiodides, 2-hydroxy-ethanesulfonates, lactates, maleates, methanesulfonates, nicotinates, 2-naphthalenesulfonates, oxalates, palmoates, pectinates, persulfates, 3-phenylpropionates, picrates, pivalates, propionates, succinates, tartrates, thiocyanates, tosylates, mesylates and undecanoates.

In one embodiment, the pharmaceutically acceptable salt is a hydrochloride salt.

Some of the compounds useful in the present invention can exist in different polymorphic forms. As known in the art, polymorphism is an ability of a compound to crystallize as more than one distinct crystalline or "polymorphic" species. A polymorph is a solid crystalline phase of a compound with at least two different arrangements or polymorphic forms of that compound molecule in the solid state. Polymorphic forms of any given compound are defined by the same chemical formula or composition and are as distinct in chemical structure as crystalline structures of two different chemical compounds. The use of such polymorphs is within the scope of the present invention.

Some of the compounds useful in the present invention can exist in different solvate forms. Solvates of the compounds of the invention may also form when solvent molecules are incorporated into the crystalline lattice structure of the compound molecule during the crystallization process. For example, suitable solvates include hydrates, e.g., monohydrates, dihydrates, sesquihydrates, and hemihydrates. The use of such solvates is within the scope of the present invention.

Furthermore, the present invention particularly relates to trans-4-{2-[4-(2,3-dichlorophenyl)-piperazin-1-yl]-ethyl}-N,N-dimethylcarbamoyl-cyclohexylamine and pharmaceutically acceptable salts thereof, more particularly to the use of trans-4-{2-[4-(2,3-dichlorophenyl)-piperazin-1-yl]-ethyl}-N,N-dimethylcarbamoyl-cyclohexylamine hydrochloride for use in a method of treating schizophrenia with the therapeutically effective amount of 1.5, 3.0 or 4.5 mg/day.

The active ingredient is administered in an amount of 1.5 mg, 3 mg or 4.5 mg per day.

The desired dose may be administered as one or more daily sub dose(s) administered at appropriate time intervals throughout the day, or alternatively, in a single dose, for example, for morning or evening administration. For example, the daily dosage may be divided into one, into two, into three, or into four divided daily doses.

The duration of the treatment may be decades, years, months, weeks, or days, as long as the benefits persist.

In one embodiment of the present invention, the therapeutically effective amount of trans-4-{2-[4-(2.3-dichlorophenyl)-piperazin-1-yl]-ethyl}-N,N-dimethylcarbamoyl-cyclohexylamine or a pharmaceutically acceptable salt thereof is 1.5 mg/day.

In another embodiment of the present invention, the therapeutically effective amount of trans-4-{2-[4-(2,3-dichlorophenyl)-piperazin-1-yl]-ethyl}-N,N-dimethylcarbamoyl-cyclohexylamine or a pharmaceutically acceptable salt thereof is 3.0 mg/day.

In a further embodiment of the present invention, the therapeutically effective amount of trans-4-{2-[4-(2,3-dichlorophenyl)-piperazin-1-yl]-ethyl}-N,N-dimethylcarbamoyl-cyclohexylamine or a pharmaceutically acceptable salt thereof is 4.5 mg/day.

In additional embodiments, the active ingredient used is trans-4-{2-[4-(2,3-dichlorophenyl)-piperazin-1-yl]-ethyl}-N,N-dimethylcarbamoyl-cyclohexylamine hydrochloride.

In one embodiment, the active ingredient is used in one or two divided daily doses.

In one embodiment, the use of the active ingredient provides therapeutic effects in the treatment of cognitive symptoms of schizophrenia. In another embodiment, the use of the active ingredient provides therapeutic effects in the treatment of positive symptoms of schizophrenia. In a further embodiment, the use of the active ingredient provides therapeutic effects in the treatment of negative symptoms of schizophrenia.

In other embodiments, the use of the active ingredient provides therapeutic effects in the treatment of affective symptoms of schizophrenia, residual symptoms of schizophrenia, or schizophreniform disorder.

The compounds of formula (I) can be used alone or as an active ingredient of a pharmaceutical composition.

Numerous standard references are available that describe procedures for preparing various formulations suitable for administering the compounds according to the invention. Examples of potential formulations and preparations are contained, for example, in the Handbook of Pharmaceutical Excipients, American Pharmaceutical Association (current edition); Pharmaceutical Dosage Forms: Tablets (Lieberman, Lachman and Schwartz, editors) current edition, published by Marcel Dekker, Inc., as well as Remington's Pharmaceutical Sciences (Arthur Osol, editor), 1553-1593 (current edition).

Suitable dosage forms include oral, rectal, sub-lingual, mucosal, nasal, ophthalmic, subcutaneous, intramuscular, intravenous, transdermal, spinal, intrathecal, intra-articular, intra-arterial, sub-arachinoid, bronchial, lymphatic, and intra-uterille administration, and other dosage forms for systemic delivery of active ingredients. Formulations suitable for oral administration are preferred.

To prepare such pharmaceutical dosage forms, the active ingredient, is intimately admixed with a pharmaceutical carrier according to conventional pharmaceutical compounding techniques. The carrier may take a wide variety of forms depending on the form of preparation desired for administration.

In preparing the compositions in oral dosage form, any of the usual pharmaceutical media may be employed. Thus, for liquid oral preparations, such as, for example, suspensions, elixirs and solutions, suitable carriers and additives include water, glycols, oils, alcohols, flavoring agents, preservatives, and coloring agents. For solid oral preparations such as, for example, powders, capsules and tablets, suitable carriers and additives include starches, sugars, diluents, granulating agents, lubricants, binders, and disintegrating agents. Due to their ease in administration, tablets and capsules represent the most advantageous oral dosage unit form. If desired, tablets may be sugar coated or enteric coated by standard techniques.

For parenteral formulations, the carrier will usually comprise sterile water, though other ingredients, for example, ingredients that aid solubility or for preservation, may be included. Injectable solutions may also be prepared in which case appropriate stabilizing agents may be employed.

In some applications, it may be advantageous to utilize the active agent in a "vectorized" form, such as by encapsulation of the active agent in a liposome or other encapsulant medium, or by fixation of the active agent, e.g., by covalent bonding, chelation, or associative coordination, on a suitable biomolecule, such as those selected from proteins, lipoproteins, glycoproteins, and polysaccharides.

Formulations suitable for oral administration may be presented as discrete units such as capsules, cachets, tablets, or lozenges, each containing a predetermined amount of the active ingredient as a powder or granules. Optionally, a suspension in an aqueous liquor or a non-aqueous liquid may be employed, such as a syrup, an elixir, an emulsion, or a draught.

A tablet may be made by compression or molding, or wet granulation, optionally with one or more accessory ingredients. Compressed tablets may be prepared by compressing in a suitable machine, with the active compound being in a free-flowing form such as a powder or granules which optionally is mixed with, for example, a binder, disintegrant, lubricant, inert diluent, surface active agent, or discharging agent. Molded tablets comprised of a mixture of the powdered active compound with a suitable carrier may be made by molding in a suitable machine.

A syrup may be made by adding the active compound to a concentrated aqueous solution of a sugar, for example sucrose, to which may also be added any accessory ingredient(s). Such accessory ingredient(s) may include flavorings, suitable preservative, agents to retard crystallization of the sugar, and agents to increase the solubility of any other ingredient, such as a polyhydroxy alcohol, for example glycerol or sorbitol.

Formulations suitable for parenteral administration usually comprise a sterile aqueous preparation of the active compound, which preferably is isotonic with the blood of the recipient (e.g., physiological saline solution). Such formulations may include suspending agents and thickening agents and liposomes or other microparticulate systems which are designed to target the compound to blood components or one or more organs. The formulations may be presented in unit-dose or multi-dose form.

Parenteral administration may comprise any suitable form of systemic delivery or delivery directly to the CNS. Administration may for example be intravenous, intra-arterial, intrathecal, intramuscular, subcutaneous, intramuscular, intra-abdominal (e.g., intraperstoneal), etc., and may be effected by infusion pumps (external or implantable) or any other suitable means appropriate to the desired administration modality.

Nasal and other mucosal spray formulations (e.g. inhalable forms) can comprise purified aqueous solutions of the active compounds with preservative agents and isotonic agents. Such formulations are preferably adjusted to a pH and isotonic state compatible with the nasal or other mucous membranes. Alternatively, they can be in the form of finely divided solid powders suspended in a gas carrier. Such formulations may be delivered by any suitable means or method, e.g., by nebulizer, atomizer, or metered dose inhaler.

Formulations for rectal administration may be presented as a suppository with a suitable carrier such as cocoa butter, hydrogenated fats, or hydrogenated fatty carboxylic acids.

Transdermal formulations may be prepared by incorporating the active agent in a thixotropic or gelatinous carrier such as a cellulosic medium, e.g., methyl cellulose or hydroxyethyl cellulose, with the resulting formulation then being packed in a transdermal device adapted to be secured in dermal contact with the skin of a wearer.

In addition to the aforementioned ingredients, formulations of this invention may further include one or more accessory ingredient(s) selected from diluents, buffers, flavoring agents, binders, disintegraats, surface active agents, thickeners, lubricants, preservatives (including antioxidants).

The formulations can have immediate release, sustained release, delayed-onset release or any other release profile known to one skilled in the art.

Trans-4-{2-[4-(2,3-dichlorophenyl)-piperazin-1-yl]-ethyl}-N,N-dimethylcarbamoyl-cyclohexylamine or a pharmaceutically acceptable salt thereof may be used as an adjunctive treatment to one or more additional therapeutic agents (e.g., antipsychotics, antidepressants). Further, trans-4-{2-[4-(2,3-dichlorophenyl)-piperazin-1-yl]-ethyl}-N,N-dimethylcarbamoyl-cyclohexylamine or a pharmaceutically acceptable salt thereof may be co-administered as a combination therapy with one or more one or more additional therapeutic agents (e.g., antipsychotics, antidepressants).

The antipsychotic may be an atypical or a typical antipsychotic, preferably an atypical antipsychotic. Examples of atypical antipsychotics include olanzapine, clozapine, risperidone, sertindole, quetiapine, aripiprazole, ziprasidone, and surmontil, Examples of typical antipsychotics include : acepromazine, benperidol, bromazepam, bromperidol, chlorpromazine, chlorprothixene, clotiapine, cyamemazine, diazepam, dixyrazine, droperidol, flupentixol, fluphenazine, fluspirilene, haloperidol, heptaminol, isopropamide iodide, levomepromazine, levosulpiride, loxapine, melperone, mesoridazine, molindone, oxypertine, oxyprothepine, penfluridal, perazine, periciazine, perphenazine, pimozide, pipamperone, pipotiazine, prochlorperazine, promazine, promethazine, prothipendyl, pyridoxine, sulpiride, sultopride, tetrabenazine, thioproperazine, thioridazine, tiapride, thiotixene, trifluoperazine, triflupromazine, trihexyphenidyl, and zuclopenthixol.

### Definitions

The term "pharmaceuticaly acceptable" means biologically or pharmacologically compatible for *in vivo* use in animals or humans, and preferably means approved by a regulatory agency of the Federal or a state government or listed in the U.S. Pharmacopeia or other generally recognized pharmacopeia for use in animals, and more particularly in humans.

The term "schizophrenia" is intended to include the group of mental disorders characterized by disruptions in thinking and perception, and includes schizophrenia (and all its subtypes; paranoid, catatonic, disorganized, residual, undifferentiated) and other psychotic disorders (as per Diagnostic and Statistical Manual for Mental Disorders, Fourth Edition, Washington, D.C (1994): American Psychiatric Association, or The ICD-10 Classification of Mental and Behavioural Disorders: Clinical Descriptions and Diagnostic Guidelines, Geneva (1992): World Health Organization) such as schizophreniform and schizoaffective disorders, or brief psychotic disorder.

In a clinical evaluation, schizophrenia is commonly marked by "positive symptoms" such as hallucinations (especially auditory hallucination which are usually experienced as voices), disorganized thought processes and delusions as well as "negative symptoms" which include affective flattening, alogia, avolition, and anhedonia.

The term "the negative symptoms of schizophrenia" refers to a class of symptoms of schizophrenia which can be considered to reflect a 'loss' in functional, directed thought or activity. Negative symptoms of schizophrenia are well known in the art, and include affective flattening (characterized by, for example, an immobile and/or unresponsive facial expression, poor eye contact and reduced body language), alogia ('poverty of speech' or brief, laconic and/or empty replies), avolition (characterized by a reduced or absent ability to initiate and carry out goal-directed activities), anhedonia (loss of interest or pleasure), asocialty (reduced social drive and interaction), apathy and other negative symptoms known to those of skill in the art. The negative symptoms of schizophrenia may be assessed using any methodology known in the art including, but not limited to, the Brief Psychiatric Rating Scale (BPRS), and the Positive and Negative Symptom Scale (PANSS). The BPRS and PANSS have subscales or factors that can be used to measure negative symptoms. Other scales have been designed to address specifically negative symptoms: For example the Scale for the Assessment of Negative Symptoms (SANS), the Negative Symptoms Assessment (NSA) and the Schedule for the Deficit Syndrome (SDS). Subscales of the BPRS and PANSS may also be used to assess positive symptoms, although methods for specifically assessing positive symptoms are also available (e.g., the Scale for the Assessment of Positive Symptoms, or SAPS).

The term "cognitive deficits associated with schizophrenia" refers to cognitive deficits in schizophrenia patients. Cognitive impairment in schizophrenia is a core feature of the illness (i.e. not a result of treatment or clinical symptoms). Cognitive deficits include, but are not limited to deficits of attention/vigilance, working memory, verbal learning and memory, visuospatial memory, reasoning/problem solving and social cognition. There are numerous neuropsychological tests used to measure cognitive deficits in schizophrenia, such as the Wisconsin Card Sorting Test (WCST).

The terms "treat," "treatment," and "treating" refer to one or more of the following:
(a) relieving or alleviating at least one symptom of a disorder in a subject;
(b) relieving or alleviating the intensity and/or duration of a manifestation of a disorder experienced by a subject including, but not limited to, those that are in response to a given stimulus;
(c) arresting, delaying the onset (i.e., the period prior to clinical manifestation of a disorder) and/or reducing the risk of developing or worsening a disorder.

An "effective amount" means the amount of an active ingredient that, when administered to a patient (e.g., a mammal) for treating a disease (i.e., schizophrenia), is sufficient to effect such treatment for the disease, or an amount that is sufficient for modulating a dopamine receptor (e.g., the dopamine D₂ and/or dopamine D₃ receptor) to achieve the objectives of the invention.

A subject or patient in whom administration of the therapeutic compound is an effective therapeutic regimen for a disease or disorder is preferably a human, but can be any animal, including a laboratory animal in the context of a trial or screening or activity experiment. Thus, as can be readily appreciated by one of ordinary skill in the art, the compounds and compositions of the present invention are particularly suited to administration to any animal, particularly a mammal, and including, but by no means limited to, humans, domestic animals, such as feline or canine subjects, farm animals, such as but not limited to bovine, equine, caprine, ovine, and porcine subjects, wild animals (whether in the wild or in a zoological garden), research animals, such as mice, rats, rabbits; goats, sheep, pigs, dogs, cats, etc., avian species, such as chickens, turkeys, songbirds, etc., i.e., for veterinary medical use.

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than I standard deviations, per practice in the art. Alternatively, "about" with respect to the compositions can mean plus or minus a range of up to 20%, preferably up to 10%, more preferably up to 5%. Alternatively, particularly with respect to biological systems or processes, the term can mean within an order of magnitude, preferably within 5-fold, and more preferably within 2-fold, of a value. Where particular values are described in the application and claims, unless otherwise stated the term "about" means within an acceptable error range for the particular value.

### EXAMPLES

### EXAMPLE 1

This clinical study will be conducted as a multicenter, randomized, double-blind, placebo-controlled, parallel-group, flexible-dose study. A total of approximately 375 inpatients patients will be selected using criteria that includes patients who (i) currently meet or have met in the past the *Diagnostic and Statistical Manual of Mental Disorders, Fourth Edition, Text Revision (DSM-IV-TR)* criteria for schizophrenia (295.30 Paranoid Type, 295.10 Disorganized Type, 295.20 Catatonic Type, or 295.90 Undifferentiated Type) based on the Structured Clinical Interview for DSM-IV (SCID), (ii) have a PANSS total score ≥ 70 and ≤ 120 at Visit 1 and at Visit 2, (iii) have a score ≥ 4 (moderate) on item P1 (delusions) or P3 (hallucinatory behavior) of the PANSS at Visit 1 and at Visit 2, and (iv) have a score ≥ 4 (moderate) on item P2 (conceptual disorganization) or P6 (suspiciousness/persecution) of the PANSS at Visit 1 and at Visit 2.

This study will be 10 weeks in duration; 6-weeks double-blind treatment and 4-weeks safety follow-up. A no-drug washout period of up to 7 days will precede randomization. Patients will be hospitalized during the screening phase. Patients will remain hospitalized for a minimum of 21 days following randomization and initiation of double-blind medication. The evaluation schedule is shown in Table 1.

**Table 1: Evaluation Schedule**

| | **Screening** | **Baseline** | **Double-Blind Phase** | | | | | | **Safety Follow-up** | |
|---|---|---|---|---|---|---|---|---|---|---|
| | **Visit 1** | **Visit 2** | **Visit 3** | **Visit 4** | **Visit 5¹** | **Visit 6¹** | **Visit 7¹** | **Visit 8²** | **Visit 9** | **Visit 10** |
| End of Study Week | -1 | 0 | 1 | 2 | 3 | 4 | 5 | 6 | 8 | 10 |
| Days | up to -7 | 0 | 7 | 14 | 21 | 28 | 35 | 42 | 56 | 70 |

Patients who meet all eligibility criteria at Visit 1 will enter a no-drug washout period of up to 7 days. Following the wash-out period, patients who meet all eligibility criteria will be assigned a randomization number at Visit 2 and dispensed the corresponding blister pack of double-blind study medication for Week 1 of double-blind treatment.

All patients meeting the eligibility criteria will be randomized (1:1:1) to one of three treatment groups:
(I) placebo,
(II) 1.5 - 4.5 mg trans-4- {2-[4-(2,3-dicbiorophanyl)-piperazin-I -yl]-ethyl}-N,N-diimethylcarbamoyl-cyclohexylamine hydrochloride, or
(III) 6 - 12 mg trans-4-{2-[4-(2,3-dichlorophenyl)-piperazin-1-yl]-ethyl}-N,N-dimethylcarbamoyl-cyclohexylamine hydrochloride (Reference)

Patients will be supplied with identically appearing capsules containing 1.5 mg, 3.0 mg, or 6.0 mg of trans-4-{2-[4-(2,3-dichlorophenyl)-piperzin-1-yl]-ethyl}-N,N-dimethylcarbamoyl-cyclohexylamine hydrochloride or placebo.

All study drugs will be dispensed in blister packs, one for each week. Each card will contain 30 capsules arranged in 10 columns and 3 rows, adequate for the 7 days of the week plus 3 extra days. The configuration of the blister pack is provided in Table 2. All study drugs will be administered as a single daily dose at bedtime. The dosing can be switched to morning if there are tolerability problems; however, any switch must allow at least 24 hours between two consecutive doses.

**Table 2: Double Blind Study Dosing Regimen**

| **Treatment Group 1 : 1.5-4.5 mg** | | | | | | | | | | |
|---|---|---|---|---|---|---|---|---|---|---|
| | **Day 1** | **Day 2** | **Day 3** | **Day 4** | **Day 5** | **Day 6** | **Day 7** | **Day 8** | **Days 9-14** | **Days 15-42** |
| Row 1 | 1.5 | 1.5 | 1.5 | 1.5 | 1.5 | 1.5 | 1.5 | 1.5 | 1.5 | 1.5 |
| Row 2 | 0 | 0 | 1.5 | 1.5 | 1.5 | 1.5 | 1.5 | 1.5 | 1.5 | 1.5 |
| Row 3 | 0 | 0 | 0 | 0 | 1.5 | 1.5 | 1.5 | 1.5 | 1.5 | 1.5 |

| **Treatment Group 2 : 6-12 mg** | | | | | | | | | | |
|---|---|---|---|---|---|---|---|---|---|---|
| | **Day 1** | **Day 2** | **Day 3** | **Day 4** | **Day 5** | **Day 6** | **Day 7** | **Day 8** | **Days 9-14** | **Days 15-42** |
| Row 1 | 1.5 | 1.5 | 3 | 3 | 3 | 3 | 3 | 3 | 6 | 6 |
| Row 2 | 0 | 0 | 0 | 0 | 0 | 0 | 3 | 3 | 3 | 3 |
| Row 3 | 0 | 0 | 0 | 0 | 3 | 3 | 3 | 3 | 3 | 3 |

| **Treatment Group 3 : Placebo** | | | | | | | | | | |
|---|---|---|---|---|---|---|---|---|---|---|
| | **Day 1** | **Day 2** | **Day 3** | **Day 4** | **Day 5** | **Day 6** | **Day 7** | **Day 8** | **Days 9-14** | **Days 15-42** |
| Row 1 | 0 | 0 | 0 | 0 | 0 | 0 | 0 | 0 | 0 | 0 |
| Row 2 | 0 | 0 | 0 | 0 | 0 | 0 | 0 | 0 | 0 | 0 |
| Row 3 | 0 | 0 | 0 | 0 | 0 | 0 | 0 | 0 | 0 | 0 |

On day 1 and day 2, all patients will be administered one capsule from row 1 of the blister pack. On day 3, the dose can be increased to two capsules (rows 1 and 2), if response is not adequate and there are no tolerability problems. Starting on day 5, the dose can be increased by one capsule to a maximum of three capsules (rows 1, 2, and 3) depending on response and tolerability. For patients randomized to Group II (1.5-4.5 mg trans-4-{2-[4-{2,3-dichlorophenyl)-piperazin-1-yl]-ethyl}-N,N-dimethylcarbamoyl-cyclohexylamine hydrochloride), the maximum dose of 4.5 mg can be reached by day 5, whereas for patients randomized to Group III (6-12 mg trans-4-{2-[4-(2,3-dichlorophenyl)-piperazin-1yl]-ethyl}-N,N-dimethylcarbamoyl-cyclohexylamine hydrochloride), the maximum dose of 12 mg can be reached by Day 9. Any dose increases will be done in increments of one capsule.

Evaluations, to be conducted using established ratings methods, include:

| Visit | Day | Evaluation |
|---|---|---|
| 1 | up to -7 | SCI-PANSS |
| 2 | 0 | SCI-PANSS, CGI-S, CDSS |
| 3 | 7 | SCI-PANSS, CGI-S, CGI-I |
| 4 | 14 | SCI-PANSS, CGI-S, CGI-I |
| 5 | 21 | SCI-PANSS, CGI-S, CGI-I, CDSS |
| 6 | 28 | SCI-PANSS, CGI-S, CGI-I |
| 7 | 35 | SCI-PANSS, CGI-S CGI-I |
| 8 | 42 | SCI-PANSS, CGI-S, CGI-I, CDSS |

| | | |
|---|---|---|
| PANSS Total Score (see, e.g., Kay et al., Schizophr. Bull., 13, 261-76, 1987) CGI-S: Clinical Global Impressions - Severiry (see, e.g., Guy ECDEU Assessment Manual for Psychopharmacology. Rockville, Md: US Department of Health, Education, and Welfare, 218-22, Publication ADM 76-338, 1976) CGI-I: Clinical Global Impressions - Improvement (see, e.g., Guy ECDEU Assessment Manual for Psychopharmacology. Rockville, Mid: US Department of Health, Education, and Welfare, 218-22, Publication ADM 76-338, 1976) CDSS: Calgary Depression Scale for Schizophrenia (see, e.g., Addington et al., Schizophr. Res., 19, 205-12, 1996) | | |

Blood samples will be collected on days 14, 21, 28, 35, 42, 56, and 70.

It is anticipated that the aforementioned treatment regime with trans-4-{2-[4-(2,3-dichlorophenyl)-piperazin-1-yl]-ethyl}-N,N-dimethylcarbamoyl-cyclohexylamine hydrochloride will show significant effectiveness in the treatment of schizophrenia, when compared to patients treated with placebo.

## Claims

1. Trans-4-{2-[4-(2,3-dichlorophenyl)-piperazin-1-yl]-ethyl}-N,N-dimethylcarbamoyl-cyclohexylamine and/or salts and/or hydrates and/or solvates and/or polymorphs thereof for use in a method of treating schizophrenia with the therapeutically effective amount of 1.5, 3.0 or 4.5 mg/day.

2. Trans-4-{2-[4-(2,3-dichlprophenyl)-piperazin-1-yl]-ethyl}-N,N-dimethylcarbamoyl-cyclohexylamine for use according to claim 1, in the form of trans-4-{2-[4-(2,3-dichlorophenyl)-piperazin-1-yl]-ethyl}-N,N-dimethylcarbamoyl-cyclohexylamine hydrochloride and/or hydrates and/or solvates and/or polymorphs thereof.

3. Trans-4-{2-[4-(2,3-dichlorophenyl)-piperazin-l-yl]-ethyl}-N,N-dimethylcarbamoyl-cyclohexylamine for use according to claim 1 or 2, wherein the therapeutically effective amount is divided into one, two, three or four daily doses.

4. Trans-4-{2-[4-(2,3-dichlorophenyl)-piperazin-1-yl]-ethyl}-N,N-dimethylcarbamoyl-cyclohexylamine for use according to any one of claims 1-3, wherein it is for use in a method of treating cognitive symptoms of schizophrenia.

5. Trans-4-{2-[4-(2,3-dichlorophenyl)-piperazin-l-yl]-ethyl}-N,N-dimethylcarbamoyl-cyclohexylamine for use according to any one of claims 1-3, wherein it is for use in a method of treating negative symptoms of schizophrenia.

6. Trans-4-{2-[4-(2,3-dichlorophenyl)-piperazin-1-yl]-ethyl}-N,N-dimethylcarbamoyl-cyclohexylamine for use according to any one of claims 1-3, wherein it is for use in a method of treating positive symptoms of schizophrenia.

7. Trans-4-{2-[4-(2,3-dichlorophenyl)-piperazin-1-yl]-ethyl}-N,N-dimethylcarbamoyl-cyclohexylamine for use according to any one of claims 1-3, wherein it is for use in a method of treating affective and residual symptoms of schizophrenia.

8. (Trans-4-{2-[4-(2,3-dichlorophenyl)-piperazin-l-yl]-ethyl}-N,N-dimethylcarbamoyl-cyclohexylamine for use according to any one of claims 1-3, wherein it is for use in a method of treating secondary social and occupational dysfunctions of schizophrenia.

9. Trans-4-{2-[4-(2,3-dichlorophenyl)-piperazin-1-yl]-ethyl}-N,N-dimethylcarbamoyl-cyclohexylamine for use according to any one of claims 1-3, wherein it is for use in a method of treating schizophrenifonn disorder and schizoaffective disorders.

## Patentansprüche

1. Trans-4-{2-[4-(2,3-dichlorophenyl)-piperazin-1-yl]-ethyl}-N,N-dimethylcarbamoylcyclohexylamin und/oder Salze und/oder Hydrate und/oder Solvate und/oder Polymorphe davon zur Verwendung in einem Verfahren zur Behandlung von Schizophrenie mit der therapeutisch effektiven Menge von 1,5, 3,0 oder 4,5 mg/Tag.

2. Trans-4-{2-[4-(2,3-dichlorophenyl)-piperazin-1-yl]-ethyl}-N,N-dimethylcarbamoylcyclohexylamin zur Verwendung gemäß Anspruch 1, in Form des Trans-4-{2-[4-(2,3-dichlorophenyl)-piperazin-1-yl]-ethyl}-N,N-dimethylcarbamoylcyclohexylamin-Hydrochlorid und/oder Hydrate und/oder Solvate und/oder Polymorphe davon.

3. Trans-4-{2-[4-(2,3-dichlorophenyl)-piperazin-1-yl]-ethyl}-N,N-dimethylcarbamoylcyclohexylamin zur Verwendung gemäß Anspruch 1 oder 2, worin die therapeutisch wirksame Menge in eine, zwei, drei oder vier tägliche Dosen eingeteilt ist.

4. Trans-4-{2-[4-(2,3-dichlorophenyl)-piperazin-1-yl]-ethyl}-N,N-dimethylcarbamoylcyclohexylamin zur Verwendung gemäß irgendeinem der Ansprüche 1 bis 3, worin es zur Verwendung in einem Verfahren zur Behandlung kognitiver Symptome von Schizophrenie dient.

5. Trans-4-{2-[4-(2,3-dichlorophenyl)-piperazin-1-yl]-ethyl}-N,N-dimethylcarbamoylcyclohexylamin zur Verwendung gemäß irgendeinem der Ansprüche 1 bis 3, worin es zur Verwendung in einem Verfahren zur Behandlung negativer Symptome von Schizophrenie dient.

6. Trans-4-{2-[4-(2,3-dichlorophenyl)-piperazin-1-yl]-ethyl}-N,N-dimethylcarbamoylcyclohexylamin zur Verwendung gemäß irgendeinem der Ansprüche 1 bis 3, worin es zur Verwendung in einem Verfahren zur Behandlung positiver Symptome von Schizophrenie dient.

7. Trans-4-{2-[4-(2,3-dichlorophenyl)-piperazin-1-yl]-ethyl}-N,N-dimethylcarbamoylcyclohexylamin zur Verwendung gemäß irgendeinem der Ansprüche 1 bis 3, worin es zur Verwendung in einem Verfahren zur Behandlung affektiver und residualer Symptome von Schizophrenie dient.

8. Trans-4-{2-[4-(2,3-dichlorophenyl)-piperazin-1-yl]-ethyl}-N,N-dimethylcarbamoylcyclohexylamin zur Verwendung gemäß irgendeinem der Ansprüche 1 bis 3, worin es zur Verwendung in einem Verfahren zur Behandlung sekundärer sozialer und okkupationaler Fehlfunktionen von Schizophrenie dient.

9. Trans-4-{2-[4-(2,3-dichlorophenyl)-piperazin-1-yl]-ethyl}-N,N-dimethylcarbamoylcyclohexylamin zur Verwendung gemäß irgendeinem der Ansprüche 1 bis 3, worin es zur Verwendung in einem Verfahren zur Behandlung schizophrenieformer Störung und schizoaffektiver Störungen dient.

## Revendications

1. Trans-4-{2-[4-(2,3-dichlorophényl)-pipérazin-1-yl]-éthyl}-N,N-diméthylcarbamoyl-cyclohexylamine et/ou sels et/ou hydrates et/ou solvates et/ou polymorphes de celle-ci pour l'utilisation dans un procédé de traitement de la schizophrénie avec la quantité thérapeutiquement efficace de 1,5, 3,0 ou 4,5 mg/jour.

2. Trans-4-{2-[4-(2,3-dichlorophényl)-pipérazin-1-yl]-éthyl}-N,N-diméthylcarbamoyl-cyclohexylamine pour une utilisation selon la revendication 1, sous forme de chlorhydrate de trans-4-{2-[4-(2,3-dichlorophiényl)-pipérazin-1-yl]-éthyl}-N,N-diméthylcarbamoyl-cyclohexylamine et/ou d'hydrates et/ou de solvates et/ou de polymorphes de celle-ci.

3. Trans-4-{2-[4-(2,3-dichlorophényl)-pipérazin-1-yl]-éthyl}-N,N-diméthylcarbamoyl-cyclohexylamine pour une utilisation selon la revendication 1 ou 2, dans laquelle la quantité thérapeutiquement efficace se divise en une, deux, trois ou quatre doses quotidiennes.

4. Trans-4-{2-[4-(2,3-dichlorophényl)-pipérazin-1-yl]-éthyl}-N,N-diméthylcarbamoyl-cyclohexylamine pour une utilisation selon l'une quelconque des revendications 1 à 3, destinée à l'utilisation dans un procédé de traitement des symptômes cognitifs de la schizophrénie.

5. Trans-4-{2-[4-(2,3-dichlorophényl)-pipérazin-1-yl]-éthyl}-N,N-diméthylcarbamoyl-cyclohexylamine pour une utilisation selon l'une quelconque des revendications 1 à 3, destinée à l'utilisation dans un procédé de traitement des symptômes négatifs de la schizophrénie.

6. Trans-4-{2-[4-(2,3-dichlorophényl)-pipérazin-1-yl]-éthyl}-N,N-diméthylcarbamoyl-cyclohexylamine pour une utilisation selon l'une quelconque des revendications 1 à 3, destinée à l'utilisation dans un procédé de traitement des symptômes positifs de la schizophrénie.

7. Trans-4-{2-[4-(2,3-dichlorophényl)-pipérazin-1-yl]-éthyl}-N,N-diméthylcarbamoyl-cyclohexylamine pour une utilisation selon l'une quelconque des revendications 1 à 3, destinée à l'utilisation dans un procédé de traitement des symptômes affectifs et résiduels de la schizophrénie.

8. Trans-4-{2-[4-(2,3-dichlorophényl)-pipérazin-1-yl]-éthyl}-N,N-diméthylcarbamoyl-cyclohexylamine pour une utilisation selon l'une quelconque des revendications 1 à 3, destinée à l'utilisation dans un procédé de traitement des dysfonctionnements sociaux et professionnels secondaires de la schizophrénie.

9. Trans-4-{2-[4-(2,3-dichlorophényl)-pipérazin-1-yl]-éthyl}-N,N-diméthylcarbamoyl-cyclohexylamine pour une utilisation selon l'une quelconque des revendications 1 à 3, destinée à l'utilisation dans un procédé de traitement du trouble schizophréniforme et des troubles schizo-affectifs.
